# EUROPEAN PATENT SPECIFICATION

(11) **EP 4 146 082 B1**
(45) Date of publication and mention of the grant of the patent: **23.10.2024**
(21) Application number: 21723977.1
(22) Date of filing: 06.05.2021
(51) Int. Cl.: A61B 8/08, A61B 5/107, A61B 8/00

(54) **BOUNDARY CORRECTION IN ULTRASOUND IMAGING AND ASSOCIATED DEVICES, SYSTEMS, AND METHODS**
GRENZKORREKTUR IN DER ULTRASCHALLBILDGEBUNG UND ZUGEHÖRIGE VORRICHTUNGEN, SYSTEME UND VERFAHREN
CORRECTION DE LIMITE DANS L'IMAGERIE PAR ULTRASONS ET DISPOSITIFS, SYSTÈMES ET PROCÉDÉS ASSOCIÉS

(30) Priority: 08.05.2020 US 202063021923 P
(43) Date of publication of application: 15.03.2023
(73) Proprietor: Koninklijke Philips N.V., 5656 AG Eindhoven (NL)
(72) Inventor: SETTLEMIER, Scott, Holland, 5656 AE Eindhoven (NL); SALGO, Ivan, 5656 AE Eindhoven (NL); PRATER, David, 5656 AE Eindhoven (NL)
(74) Representative: Philips Intellectual Property & Standards
(86) International application number: PCT/EP2021/061936
(87) International publication number: WO 2021/224365

(56) References cited:
- US-A1- 2011 098 565
- US-A1- 2018 357 770
- US-B2- 10 426 430

## Description

### TECHNICAL FIELD

The present disclosure relates generally to systems for ultrasound imaging. In particular, ultrasound imaging systems can determine and apply a correction vector to boundaries within an ultrasound image to correct inaccuracies resulting blurring caused by spatial variation in ultrasound response.

### BACKGROUND

Ultrasound imaging systems are widely used for medical imaging. For example, a medical ultrasound system may include an ultrasound transducer probe coupled to a processing system and one or more display devices. The ultrasound transducer probe may include an array of ultrasound transducer elements that transmit acoustic waves into a patient's body and record acoustic waves reflected from the internal anatomical structures within the patient's body, which may include tissues, blood vessels, and internal organs. The transmission of the acoustic waves and/or the reception of reflected acoustic waves or echo responses can be performed by the same set of ultrasound transducer elements or different sets of ultrasound transducer elements. The processing system can apply beamforming, signal processing, and/or imaging processing to the received echo responses to create an image of the patient's internal anatomical structures.

Ultrasound imaging is a safe, useful, and in some applications, non-invasive tool for diagnostic examination, interventions, and/or treatment. Ultrasound imaging can provide insights into an anatomy before a surgery or other major procedure is performed as well as monitor and/or track changes to a particular anatomical feature over time. Many ultrasound imaging systems capture and/or calculate dimensions of anatomical structures during an ultrasound examination.

Ultrasound imaging systems may be modelled as the convolution of a spatially varying point spread function having a Gaussian blurring effect which increases with depth or distance from the ultrasound transducer probe. The blurring effect of the ultrasound point spread function may result in significant inaccuracies when calculating dimensions of anatomical structures within a patient. For example, ultrasound imaging systems tend to underestimate volumes and other dimensions of hypoechoic chambers such as ventricles, atria, or cysts and overestimate hyperechoic regions. Efforts to address these inaccuracies, such as with deconvolution are unsatisfactory. For example, deconvolution of ultrasound imaging is generally difficult and impractical for timely quantification. Other ultrasound imaging systems may apply a constant offset to calculated boundaries to mitigate the inaccuracies introduced by the point spread function, but the spatially varying nature of the point spread function blurring effect makes such a solution crude and inaccurate.

In US 2018/357770 A1, it is disclosed a method for determining a contour of an organ in an ultrasound image that includes detecting an organ within the ultrasound image, obtaining a centroid position for the organ, extending a set of radial lines from the centroid position beyond an expected organ boundary, determining cost values at candidate nodes on each radial line, of the set of radial lines, by applying costs along gradient vectors that are normal to a contour between adjacent nodes on different radial lines, and selecting a final organ boundary contour based on a cost function analysis of paths through the candidate nodes.

### SUMMARY

Embodiments of the present disclosure are systems, devices, and methods for ultrasound imaging that provide more accurate representation of anatomy in ultrasound images by correcting inaccuracies resulting from the ultrasound point spread function. For example, the present disclosure includes calculating and applying a correction vector to boundaries of anatomical structures imaged in a patient's anatomy.

The ultrasound imaging system described herein may receive and/or calibrate a constant value corresponding to characteristics of an anatomical structure to be imaged. These characteristics may include acoustic impedance of materials in or around the anatomical structure. The system may image the anatomical structure and use the received and/or calibrated value to calculate a vector corresponding to the direction and distance between a measured boundary of the anatomical structure and the actual boundary of the anatomical structure. This correction vector may vary depending on the orientation or skew angle of a boundary of an anatomical structure with respect to the ultrasound imaging beam and the magnitude of the ultrasound point spread function blurring effect varying with depth at the location. The ultrasound imaging system may then apply the appropriate correction vector to any location along a boundary of an anatomical structure to correct for any inaccuracies in volume or other dimension of the anatomical structure.

The constant value received and/or calibrated by the ultrasound imaging system may be calibrated before an imaging procedure is performed. An ultrasound phantom of a known volume having similar characteristics including acoustic impedance, volume, overall shape, or other characteristics, may be used to calibrate the constant. Least squares fitting may be used by the ultrasound imaging system to determine the most accurate value of the constant to be applied for anatomical structures similar to the phantom used. Other forms of regression analysis may similarly be employed.

The ultrasound imaging system may display to a user metrics related to the dimensions of the anatomical structure being imaged. These metrics may include measurements made before and after a correction vector is applied to an image. The ultrasound imaging system may further display to a user one or more ultrasound images or videos. Displayed ultrasound images or videos may comprise lines or other graphical representations generated and overlaid on the image or video representing boundaries of an anatomical structure as calculated by the ultrasound imaging system.

In an exemplary aspect, an ultrasound imaging system comprises a processor circuit configured for communication with an ultrasound probe, the processor circuit configured to: receive, from the ultrasound probe, ultrasound data representative of an ultrasound beam imaging an anatomical structure; determine, based on the ultrasound data, a measured boundary of the anatomical structure, wherein the measured boundary includes a plurality of locations; determine a plurality of correction vectors corresponding to the plurality of locations of the measured boundary, wherein a magnitude of a respective correction vector is based on at least one of: a depth of a corresponding location relative to the ultrasound probe; or an orientation of the measured boundary at the corresponding location relative to the ultrasound beam; apply the plurality of correction vectors to the plurality of locations of the measured boundary to determine a corrected boundary; and output, to a display in communication with the processor circuit, an ultrasound image based on the ultrasound data, wherein the ultrasound image includes the corrected boundary.

In some aspects, a direction of the plurality of correction vectors is at least one of normal to the measured boundary or normal to the corrected boundary. The plurality of correction vectors are configured to correct an effect of a point spread function of the ultrasound imaging system. In some aspects, the processor circuit is configured to model the point spread function as a Gaussian function. In some aspects, the magnitude of the respective correction vector is based on the depth of the corresponding location relative to the ultrasound probe and the orientation of the measured boundary at the corresponding location relative to the ultrasound beam. In some aspects, the magnitude of the respective correction vector, for a given orientation of the measured boundary at the corresponding location, is larger when the corresponding location is at a larger depth relative to the ultrasound probe and smaller when the corresponding location is at a smaller depth relative to the ultrasound probe. In some aspects, the magnitude of the respective correction vector, for a given depth of the corresponding location relative to the ultrasound probe, is larger when the orientation of the measured boundary is parallel to the ultrasound beam and smaller when the orientation of the measured boundary is perpendicular to the ultrasound beam. In some aspects, the plurality of correction vectors is further based on a calibrated value corresponding to one or more characteristics of the anatomical structure. In some aspects, the processor circuit is further configured to calculate, based on the corrected boundary, a metric associated with the anatomical structure. In some aspects, the processor circuit is configured to output the calculated metric to the display. In some aspects, the metric comprises a volume of the anatomical structure. In some aspects, the processor circuit is further configured to output the measured boundary to the display. In some aspects, the corrected boundary comprises a graphical overlay on the ultrasound image. In some aspects, a direction of the plurality of correction vectors is: inward relative to the measured boundary when the anatomical structure comprises a hyperechoic chamber; and outward relative to the measured boundary when the anatomical structure comprises a hypoechoic chamber. In some aspects, the system further comprises the ultrasound probe.

In an exemplary aspect, an ultrasound imaging method comprises receiving, at a processor circuit in communication with an ultrasound probe, ultrasound data representative of an ultrasound beam imaging an anatomical structure; determining, by the processor circuit, a measured boundary of the anatomical structure based on the ultrasound data, wherein the measured boundary includes a plurality of locations; determining, by the processor circuit, a plurality of correction vectors corresponding to the plurality of locations of the measured boundary wherein the plurality of correction vectors are configured to correct an effect of a point spread function of the ultrasound imaging system, wherein a magnitude of a respective correction vector is based on at least one of: a depth of a corresponding location relative to the ultrasound probe; or an orientation of the measured boundary at the corresponding location relative to the ultrasound beam; applying, by the processor circuit, the plurality of correction vectors to the plurality of locations of the measured boundary to determine a corrected boundary; and outputting, to a display in communication with the processor circuit, an ultrasound image based on the ultrasound data, wherein the ultrasound image includes the corrected boundary.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a schematic diagram of an ultrasound imaging system, according to aspects of the present disclosure.
Fig. 2 is a schematic diagram of a processor circuit, according to aspects of the present disclosure.
Fig. 3 is a diagrammatic view of an anatomical boundary defined by at least two media within a patient, according to aspects of the present disclosure.
Fig. 4 is a diagrammatic view of an anatomical boundary within a patient as measured by an ultrasound imaging system, according to aspects of the present disclosure.
Fig. 5 is a diagrammatic view of an anatomical region within a patient as measured by an ultrasound imaging system and illustrating an actual boundary and a measured boundary, according to aspects of the present disclosure.
Fig. 6 is a diagrammatic view of an anatomical boundary within a patient as measured by an ultrasound imaging system and illustrating a correction of the difference between an actual boundary and a measured boundary, according to aspects of the present disclosure.
Fig. 7 is a diagrammatic view of an anatomical structure within a patient as measured by an ultrasound imaging system and illustrating the difference between an actual boundary and a measured boundary, according to aspects of the present disclosure.
Fig. 8 is a diagrammatic view of a screen display including an ultrasound image generated by an ultrasound imaging system, according to aspects of the present disclosure.
Fig. 9 is a diagrammatic view of an enlarged portion of the ultrasound image of Fig. 8, according to aspects of the present disclosure.
Fig. 10 is a diagrammatic view of a screen display including an ultrasound image generated by the ultrasound imaging system, according to aspects of the present disclosure.
Fig. 11 is a flow diagram of a method employed by an ultrasound imaging system to correct inaccuracies in an ultrasound image, according to aspects of the present disclosure.
Fig. 12 is a diagrammatic view of an anatomical structure within a patient displayed in cartesian space, according to aspects of the present disclosure.
Fig. 13 is a diagrammatic view of the anatomical structure of Fig. 12 as measured by an ultrasound imaging system, according to aspects of the present disclosure.
Fig. 14 is a diagrammatic view of the anatomical structure of Fig. 12 displayed in polar space, according to aspects of the present disclosure.
Fig. 15 is a diagrammatic view of the anatomical structure of Fig. 12, as measured by an ultrasound imaging system and displayed in polar space, according to aspects of the present disclosure.
Fig. 16 is a diagrammatic view of an anatomical structure within a patient displayed in cartesian space, according to aspects of the present disclosure.
Fig. 17 is a diagrammatic view of the anatomical structure of Fig. 16, as measured by ultrasound imaging system and displayed in cartesian space, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the focusing system is described in terms of cardiovascular imaging, it is understood that it is not intended to be limited to this application. The system is equally well suited to any application requiring imaging within a confined cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

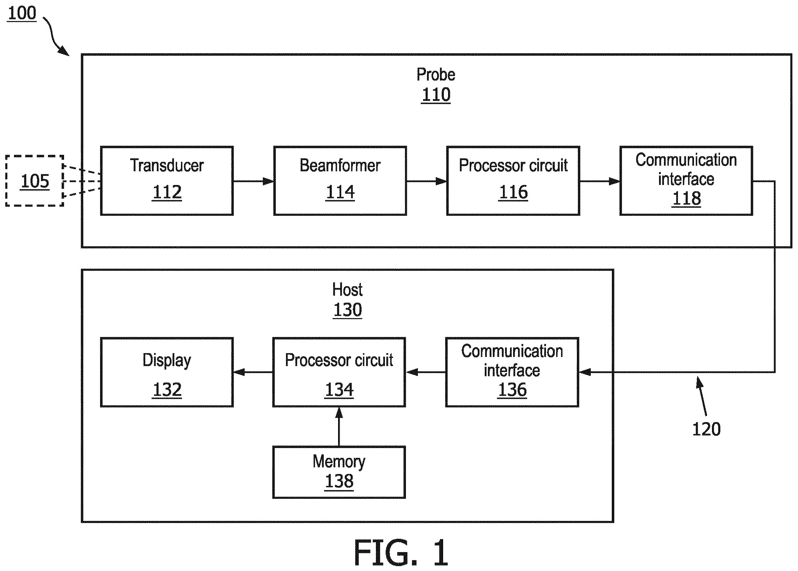

FIG. 1 is a schematic diagram of an ultrasound imaging system 100, according to aspects of the present disclosure. The system 100 is used for scanning an area or volume of a patient's body. The system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, and a communication interface 136.

Probe 110 may be in any suitable form for any suitable ultrasound imaging application including both external and internal ultrasound imaging. In some embodiments, the probe 110 is an external ultrasound imaging device including a housing configured for handheld operation by a user. The transducer array 112 can be configured to obtain ultrasound data while the user grasps the housing of the probe 110 such that the transducer array 112 is positioned adjacent to and/or in contact with a patient's skin. The probe 110 is configured to obtain ultrasound data of anatomy within the patient's body while the probe 110 is positioned outside of the patient's body. In some embodiment, the probe 110 can be an external ultrasound probe, such as a transthoracic echocardiography (TTE) probe.

In other embodiments, the probe 110 can be an internal ultrasound imaging device and may comprise a housing configured to be positioned within a lumen of a patient's body, including the patient's esophagus, heart chamber, coronary vasculature, peripheral vasculature, or other body lumen. In some embodiments, the probe 110 may be an intravascular ultrasound (IVUS) imaging catheter, or an intracardiac echocardiography (ICE) catheter. In other embodiments, probe 110 may be a transesophageal echocardiography (TEE) probe.

The transducer array 112 emits ultrasound signals towards an anatomical object 105 of a patient and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 712 acoustic elements, 1000 acoustic elements, 3000 acoustic elements, 7000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a patient's anatomy. In some embodiments, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy, such as blood vessels, nerve fibers, airways, mitral leaflets, cardiac structure, abdominal tissue structure, appendix, large intestine (or colon), small intestine, kidney, liver, and/or any other anatomy of a patient. In some aspects, the object 105 may include at least a portion of a patient's large intestine, small intestine, cecum pouch, appendix, terminal ileum, liver, epigastrium, and/or psoas muscle. The present disclosure can be implemented in the context of any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood vessels, blood, chambers or other parts of the heart, abdominal organs, and/or other systems of the body. In some embodiments, the object 105 may include malignancies such as tumors, cysts, lesions, hemorrhages, or blood pools within any part of human anatomy. The anatomy may be a blood vessel, such as an artery or a vein of a patient's vascular system, including cardiac vasculature, peripheral vasculature, neural vasculature, renal vasculature, and/or any other suitable lumen inside the body. The anatomical object 105 may additionally include ventricles or atria. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some embodiments, beamformer 114 may apply a time-delay to signals sent to individual acoustic transducers within an array in transducer 112 such that an acoustic signal is steered in any suitable direction propagating away from probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some embodiments, the transducer array 112 in combination with the beamformer 114 may be referred to as an ultrasound imaging component.

The processor circuit 116 is coupled to the beamformer 114. The processor circuit 116 may also be described as a processor circuit or processor. Processor circuit 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor circuit 134 may also be implemented as a combination of computing devices, e.g., a combination of a DSP and a microprocessor, a plurality of microprocessors, one or more microprocessors in conjunction with a DSP core, or any other such configuration. The processor circuit 116 is configured to process the beamformed image signals. For example, the processor circuit 116 may perform filtering and/or quadrature demodulation to condition the image signals. The processor circuit 116 and/or 134 can be configured to control the array 112 to obtain ultrasound data associated with the object 105.

The communication interface 118 is coupled to the processor circuit 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 nay be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor circuit 134 is coupled to the communication interface 136. The processor circuit 134 may be implemented as a combination of software components and hardware components. The processor circuit 134 may include a central processing unit (CPU), a graphics processing unit (GPU), a digital signal processor (DSP), an application-specific integrated circuit (ASIC), a controller, a FPGA device, another hardware device, a firmware device, or any combination thereof configured to perform the operations described herein. The processor circuit 134 may also be implemented as a combination of computing devices, e.g., a combination of a DSP and a microprocessor, a plurality of microprocessors, one or more microprocessors in conjunction with a DSP core, or any other such configuration. The processor circuit 134 can be configured to generate image data from the image signals received from the probe 110. The processor circuit 134 can apply advanced signal processing and/or image processing techniques to the image signals. In some embodiments, the processor circuit 134 can form three-dimensional (3D) volume image from the image data. In some embodiments, the processor circuit 134 can perform real-time processing on the image data to provide a streaming video of ultrasound images of the object 105. In some aspects, the processor circuit 134 may further perform various calculations relating to a region of interest within the patient's body. These calculations may then be displayed to the sonographer or other user via display 132.

The display 132 is coupled to the processor circuit 134. The display 132 may be a monitor or any suitable display. The display 132 is configured to display the ultrasound images, image videos, and/or any imaging information of the object 105.

The host 130 may include a memory 138, which may be any suitable storage device, such as a cache memory (e.g., a cache memory of the processor circuit 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory. The memory 138 can be configured to store patient files relating to a patient's medical history, history of procedures performed, anatomical or biological features, characteristics, or medical conditions associated with a patient, computer readable instructions, such as code, software, or other application, as well as any other suitable information or data.

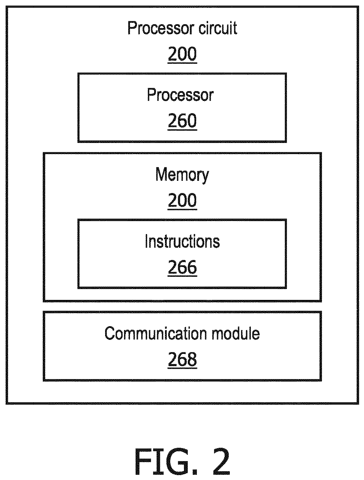

FIG. 2 is a schematic diagram of a processor circuit 200, according to embodiments of the present disclosure. The processor circuit 200 may be implemented in probe 110 and/or host 130 of FIG. 1. One or more processor circuits 200 are configured to carry out various operations described herein. As shown, the processor circuit 200 may include a processor 260, a memory 264, and a communication module 268. These elements may be in direct or indirect communication with each other, for example via one or more buses.

The processor 260 may include a CPU, a GPU, a DSP, an application-specific integrated circuit (ASIC), a controller, an FPGA, another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 264 includes a non-transitory computer-readable medium. The memory 264 may store instructions 266. The instructions 266 may include instructions that, when executed by the processor 260, cause the processor 260 to perform the operations described herein with reference to the probe 110 and/or the host 130 (FIG. 1). Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 200, the probe 110, and/or the display 132. In that regard, the communication module 268 can be an input/output (I/O) device. In some instances, the communication module 268 facilitates direct or indirect communication between various elements of the processor circuit 200 and/or the probe 110 (FIG. 1) and/or the host 130 (FIG. 1).

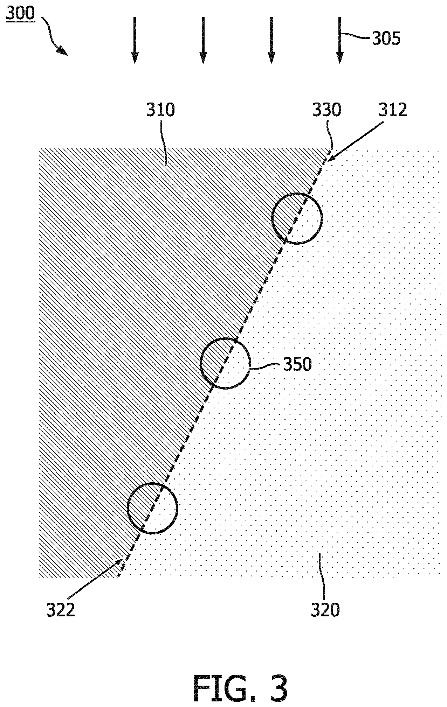

Fig. 3 is a diagrammatic view of an anatomical boundary 330 within a region 300 defined by at least two media within a patient, according to aspects of the present disclosure. Although Fig. 3 depicts a two-dimensional cross-section of the boundary 330, it is understood that the boundary 330 is a three-dimensional boundary within the patient. The boundary 330 and the cross-section of the boundary 330 can be any suitable shape. The three-dimensional boundary depicted in Fig. 3 may be of any suitable shape. The two-dimensional cross-section of the three-dimensional boundary may additionally be of any suitable shape.

A medium 310 forming the boundary 330 may be any suitable solid or semi-solid two- or three-dimensional structure, any liquid medium, or gas medium, and may be of any suitable material. For example, the medium 310 may include material of or relating to the structure of an organ, muscle, and/or other tissue in a patient's anatomy. The medium 310 may additionally include material that is substantially liquid in nature. For example, the medium 310 may include blood, blood plasma, interstitial fluid, lymph plasma, cerebrospinal fluid, intraocular fluid, serous fluid, synovial fluid, digestive fluid, urinary fluid, amniotic fluid, or any other type of suitable fluid. The medium 310 may additionally include any suitable gases found within a patient's anatomy, such as in the lungs, digestive tract, or any other location. In some embodiments, the medium 310 may include myocardium of the heart that defines a chamber within the heart. A medium 320 is also depicted in Fig. 3. The medium 320 may also include any suitable anatomical structure, liquid, or gas within a patient and may include any of the aforementioned organs, muscles, tissues, liquids, and/or gases. In some embodiments, the medium 320 may be blood within a chamber of a heart. Accordingly, an exemplary embodiment the region 300 is an interface of the heart between the myocardium and the blood inside of the heart chamber. The medium 310 and the medium 320 may exhibit different acoustic impedances such that the boundary 330 may be detected and measured by the ultrasound imaging system 100.

The boundary 330 may be defined by a surface 312 of medium 310 and/or a surface 322 of medium 320. In some embodiments, the surface 312 of medium 310 and/or the surface 322 of medium 320 may be substantially uniform. For example, surface 312 of medium 310 may be substantially continuous at a region 300 such that there are no substantially pronounced protrusions or indentations along the surface 312 of medium 310.

Region 300 may be imaged by ultrasound imaging system 100. An ultrasound imaging beam 305, depicted as downward arrows in Fig. 3, may comprise acoustic waves emitted by transducers 112 of probe 110 as described previously with reference to Fig. 1. In the embodiment of Fig. 3, ultrasound imaging beam 305 may propagate in a direction from the top of region 300 to the bottom of region 300. In general, it is understood that an ultrasound imaging beam may propagate in any suitable three-dimensional direction with respect to region 300 or any anatomical structure.

A plurality of indicators 350 are also depicted in Fig. 3. In some embodiments, indicators 350 may represent the locations at which scatterers reflect from surface 312 of medium 310 and/or surface 322 of medium 320 and may define the location of the boundary 330 within region 300. In some embodiments, the boundary 330 and/or indicators 350 are not displayed to a user in an ultrasound image on display 132 (Fig. 1). In some embodiments, a graphical representation of the boundary 330 and/or any suitable number of indicators 350 may be generated and displayed by ultrasound imaging system 100 to a user via display 132.

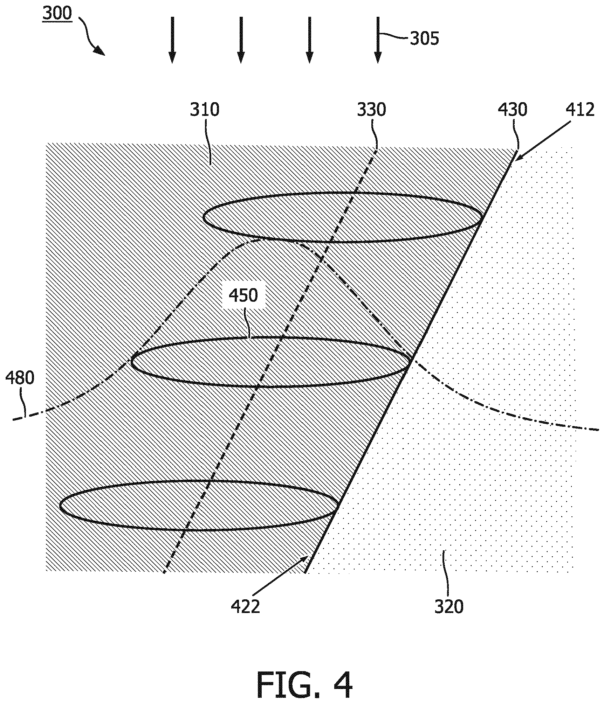

Fig. 4 is a diagrammatic view of an anatomical boundary 330 within a patient as measured by the ultrasound imaging system 100, according to aspects of the present disclosure. Fig. 4 depicts a plurality of modified indicators 450 that represent scatterers associated with the ultrasound imaging beam 305 emitted by probe 110 and/or echoes reflected from surface 312 of medium 310 and/or surface 322 of medium 320 and subsequently detected by probe 110. Fig. 4 additionally depicts a plurality of arrows also depicted in Fig. 3 representing the direction of the ultrasound imaging beam 305 emitted by probe 110.

Ultrasound imaging system 100 may create an ultrasound image for display to a user via display 132 (Fig. 1) via any suitable method. In some embodiments, ultrasound imaging system 100 may convolve a scatterer map created from acoustic signals reflected off various anatomical objects 105 received via transducers 112 within probe 110 with a multiplicative point spread function. In such embodiments, an ultrasound image created by ultrasound imaging system 100 may appear to be wider or stretched out as compared to the actual anatomical object 105 being imaged. Modified indicators 450 depict this widening effect in the form of a substantially oval shape, compared to the circular shape of the indicators 350 in Fig. 3. For example, modified indicators 450 may correspond to indicators 350 of Fig. 3, such that the location of the scatterers represented by indicators 350 are stretched by ultrasound imaging system 100 so as to be disfigured by some horizontal distance corresponding to the shape of modified indicators 450. It is noted, that the widening effect illustrated in Fig. 4 occurs only in a direction orthogonal to the direction of imaging beam 305 and in every direction orthogonal to imaging beam 305. For example, as previously mentioned, although modified indicators 450 are depicted in two dimensions or in a lateral dimension from a center point of modified indicators 450, a similar effect of equal magnitude may be observed in an elevational direction from a center point of modified indicators 450 (e.g., into and out of the plane of the page), such that the aforementioned scatterers are depicted as spread in every direction orthogonal to the direction of imaging beam 305 extending both horizontally to the left and right in both directions as shown two dimensionally as well as three dimensionally.

At least one of the consequences of the aforementioned stretching effect produced by convolving a received scatterer map with a point spread function is the boundary 330 between medium 310 and medium 320 also appears to be displaced by a corresponding distance orthogonal to the direction of imaging beam 305 within an ultrasound image generated by ultrasound imaging system 100. Fig. 4, therefore, depicts an additional boundary line corresponding to measured boundary 430 separating medium 310 and medium 320. Measured boundary 430 does not depict the accurate location of the actual boundary 330 between medium 310 and medium 320. Such a discrepancy may result in erroneous calculations of anatomical areas and/or volumes, and subsequently flow rates, efficiencies, and any number of other metrics measured or calculated based on measurements from ultrasound imaging system 100.

Curve 480 is also depicted in Fig. 4 representing the Gaussian nature of the distribution of scatterers within a convolved scatterer map used to create an ultrasound image frame. The Gaussian spread depicted by curve 480 may substantially correlate to the extent or magnitude of stretching shown by modified indicators 450 that occurs during an imaging process.

Also depicted in Fig. 4 is a measured surface 412 of medium 310 as measured by ultrasound imaging system 100. The location of measured surface 412 is not the same as the location of actual surface 312 of medium 320 in Fig. 3 due to the spreading effect mentioned previously. Additionally, measured surface 422 of medium 320 is depicted in Fig. 4. Similarly, the location of measured surface 422 is not the same as the location of the actual surface 322 of medium 320 in Fig. 3.

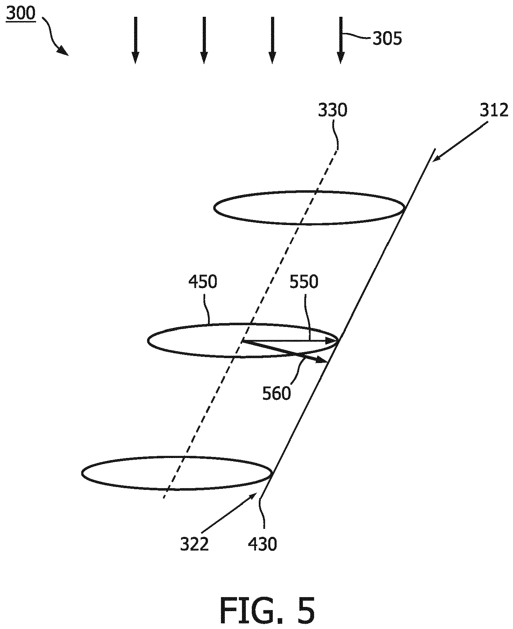

Fig. 5 is a schematic diagram of the anatomical region 300 within the patient as measured by the ultrasound imaging system 100, illustrating the difference between the actual boundary 330 and the measured boundary 430, according to aspects of the present disclosure. As previously mentioned, due to the spreading effect inherent in ultrasound imaging via convolving scatterer maps with point spread functions, each element of an ultrasound image may be displaced at some distance orthogonal to the direction of an ultrasound imaging beam 305. In Fig. 5, the measured boundary 430 is depicted displaced at a distance corresponding to the magnitude of vector 550 from actual boundary 330. While the direction of vector 550 will be in a direction orthogonal to the direction of ultrasound imaging beam 305, the particular direction of the vector 550 to the right in Fig. 5 is exemplary. It is understood that the vector 550 may extend in any direction orthogonal to ultrasound imaging beam 305, including to the left (opposite to the direction of vector 550 in Fig. 5), into the page, or out of the page.

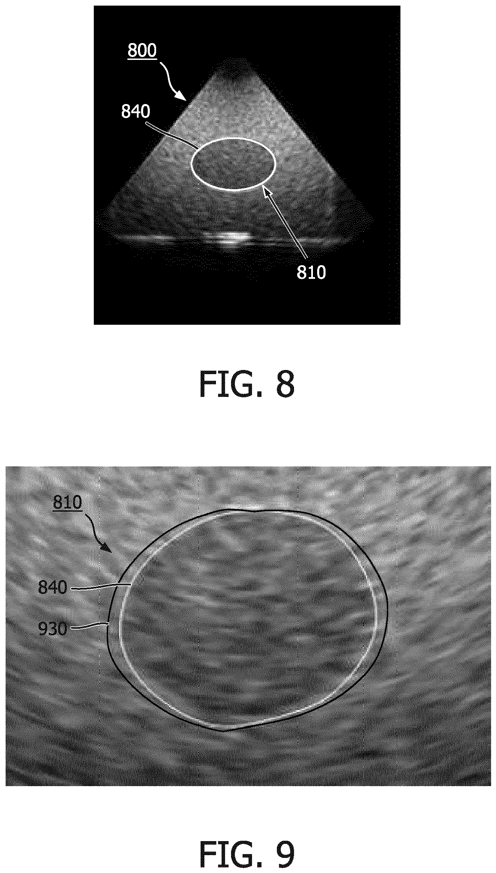

The magnitude of vector 550 may be dependent on a calibrated value corresponding to the characteristics of medium 310 (Fig. 3) and medium 320 (Fig. 3) as will be discussed in more detail hereafter and in reference to Fig. 8, Fig. 9, and Fig. 10. However, the magnitude of vector 550 may be approximated to vary with distance from probe 110 linearly. In other embodiments, other relationships between the magnitude of vector 550 and distance from probe 110 may additionally be used to approximate the appropriate magnitude of vector 550 at various locations from probe 110 within a patient's anatomy.

Displacement vector 560 represents a distance between actual boundary 330 and measured boundary 430 in a direction normal to the surface 312 of medium 310 and/or the surface 322 or medium 320 (Fig. 3). Displacement vector 560 need not be oriented orthogonal to the direction of ultrasound imaging beam 305 like vector 550 as the direction of displacement vector 560 is dictated by the orthogonal, or in other words, perpendicular, or normal, direction of surface 312 of medium 310 and/or surface 322 of medium 320. The magnitude of displacement vector 560 may represent the extent to which the spreading effect caused by convolving a scatterer map with a point spread function applies in a direction normal to the boundary 330 and represents an approximation of the distance the measured boundary 430 varies from the actual boundary 330. Displacement vector 560 may be calculated as (*N* · *D*)*N*, wherein *D* represents vector 550 and *N* represents a surface normal vector of boundary 330.

In Fig. 5, measured boundary 430 is depicted to the right of actual boundary 330 such that measured boundary 430 appears to a user of ultrasound imaging system 100 to be farther away from probe 110 than actual boundary 330. However, in other embodiments, measured boundary 430 may be depicted to the left of actual boundary 330, such that measured boundary 430 appears to a user of ultrasound imaging system 100 to be closer to the probe 110 than actual boundary 330. The location of measured boundary 430 depends primarily on the echogenicity or other characteristics of the anatomical object measured, such as the acoustic impedance, of medium 310 and medium 320 (Figs. 3 and 4). When the boundary 330 is between muscle and blood, the measured boundary 430 may be offset in the direction of the blood. Ultrasound imaging system 100 may successfully calculate vector 550 and/or displacement vector 560 in either of the aforementioned situations, including where measured boundary 430 is measured as being closer to probe 110 than actual boundary 330 or vice versa.

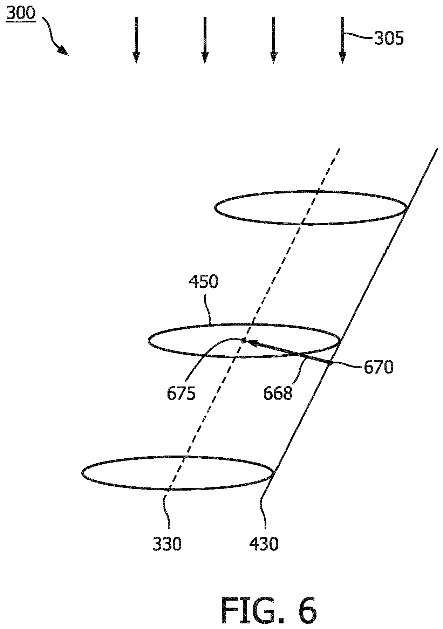

Fig. 6 is a diagrammatic view of the anatomical region 300 within the patient as measured by the ultrasound imaging system 100, illustrating a correction of the difference between the actual boundary 330 and the measured boundary 430, according to aspects of the present disclosure. Fig. 6 depicts correction vector 668, which represents the distance and direction from a point 670 on the measured boundary 430 to a point 675 on actual boundary 330. The correction vector 668 is normal to measured boundary 430 and/or the actual boundary 330. The correction vector 668 may be approximated as a vector of equal magnitude but opposite direction to displacement vector 560 (Fig. 5). For example, correction vector 668 may be approximated as - (*N* · *D)N* , *N*(-*D* · *N*), or any equivalent variation, where *D* represents vector 550 and *N* represents a surface normal vector of boundary 330. Point 670 may be any suitable position or location on surface 412 of medium 310 and/or surface 422 of medium 320. In addition, point 675 may be any corresponding point such that the correction vector 668 is normal to boundary 430 and/or boundary 330 approximately at or along surface 312 of medium 310 and/or surface 322 of medium 320. It is noted that the correction vector 668, in many cases, is only an approximation of the magnitude and direction of displacement between the measured boundary 430 and the actual boundary 330. This approximation is based on the assumption that the vectors 550 and 560 are substantially the same at the points 670 and 675. At the scales of the magnitude of the vector 550, this requirement is met. In addition, the chamber surfaces at boundary 330 are in most cases comparatively flat on the order of the point spread function effect. It is additionally noted that although only one correction vector 668 is depicted in Fig. 6 between point 670 and point 675, any suitable number of correction vectors 668 may be calculated or approximated by the ultrasound imaging system 100 at any point along a boundary of the anatomical structure to be measured.

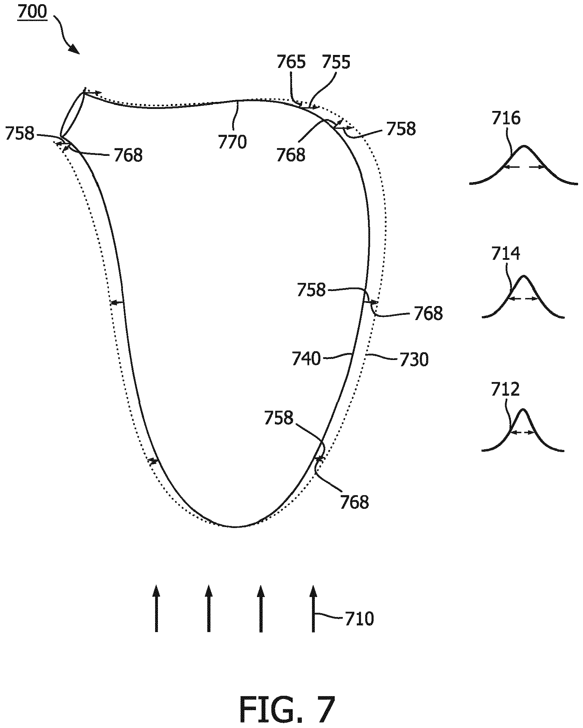

Fig. 7 is a diagrammatic view of an anatomical structure 700 within a patient as measured by the ultrasound imaging system 100, illustrating the difference in location between an actual boundary 730 and a measured boundary 740, according to aspects of the present disclosure. Anatomical structure 700 may be any suitable organ, muscle, tissue, and/or natural/man-made structure within a patient's anatomy. In an exemplary embodiment, anatomical structure 700 is a ventricle within a heart. As illustrated in Fig. 7, anatomical structure 700 may be imaged by the ultrasound imaging system 100 which emits an imaging beam 710, the direction of which is indicated with corresponding arrows. As previously mentioned, the spreading effect resulting from convolving a scatterer map with a point spread function increases with depth, or distance from the ultrasound probe. To illustrate the effect, a plurality of Gaussian curves is depicted adjacent to anatomical structure 700. The Gaussian curves 712, 714, and 716 illustrate the lateral component of the ultrasound point spread function as it is modelled across the acquisition space as PSF(x) as a Gaussian curve with a σ varying across space. Gaussian curve 712 positioned in closest proximity to the source of the imaging beam 710 (e.g., the ultrasound probe 110 of Fig. 1) illustrates a less significant spreading effect than either Gaussian curve 714 or Gaussian curve 716. Gaussian curve 714, which is positioned at a distance farther away from the probe than Gaussian curve 712 and closer to probe than Gaussian curve 716, illustrates a spreading effect greater than Gaussian curve 712 and less than Gaussian curve 716. Subsequently, Gaussian curve 716 positioned at a point furthest away from probe illustrates a more significant spreading effect than either Gaussian curve 712 or Gaussian curve 714. This point spread effect is only observed in a lateral direction (left/right in Fig. 7) or an orthogonal direction (into and out of the page in Fig. 7) to the direction of the imaging beam 710. Stated differently, the Gaussian curve corresponding to the point spread function is of greater magnitude at points farther from the ultrasound probe 110. For example, the magnitude of the Gaussian function, and subsequently the correction vector, for a given orientation of the measured boundary relative to the ultrasound probe is larger when the location is at a larger depth from the ultrasound probe and smaller when the location is at a smaller depth from the ultrasound probe.

Gaussian curve 712, Gaussian curve 714, and Gaussian curve 716 and their position with respect to the schematic diagram of anatomical structure 700 in Fig. 7 together illustrate the depth dependent nature of the point spread function observed in some embodiments of the present disclosure. In some embodiments, the point spread function may be modelled as a function, *PSF(x).* Variable *x* may be defined as a distance of a point within or around anatomical structure 700 from the source of the ultrasound beam 710 (the ultrasound probe 110). *PSF(x)* and/or *x* may include or account for any suitable offset, constant, or additional function associated with the ultrasound probe 110, anatomical structure 700, patient anatomy, and/or any other feature or characteristic of a particular application of the present disclosure. As shown by Gaussian curve 712, Gaussian curve 714, and Gaussian curve 716, the effect of *PSF(x)* is to substantially to widen the Gaussian curve associated with *x* as *x* increases. The Gaussian curve modelled as *PSF(x)* quantifies only the lateral and/or orthogonal component of the ultrasound point spread function.

Two boundary lines are also depicted in Fig. 7: actual boundary 730 and measured boundary 740. Actual boundary 730 may be substantially similar to actual boundary 330 depicted in previously presented figures. In addition, measured boundary 740 may be substantially similar to measured boundary 430 also depicted in previously presented figures. Due to the ultrasound point spread function, a measured boundary in a hypoechoic chamber similar to anatomical structure 700 (e.g., ventricles, atria, cysts, and/or other chambers with echogenic exteriors) is blurred to appear farther into the chamber than it really is in the anatomy of the patient. In other words, the ultrasound imaging system 100 may tend to underestimate volume measurements of hypoechoic chambers. When hyperechoic regions are measured by ultrasound imaging system 100, the opposite effect is observed. Stated differently, for hypoechoic chambers, like the one shown in Fig. 7, the displacement vectors extend from the actual boundary 730 inward to the measured boundary 740 and the correction vectors 768 shown in Fig. 7 extend in an opposite outward direction from the measured boundary 740 outward to the actual boundary 730. By contrast, for hyperechoic chambers the opposite is true. The displacement vectors would extend from the actual boundary outward to the measured boundary and the correction vectors would extend in an inward direction from the measured boundary back to the actual boundary.

Additionally, illustrated in Fig. 7 are a plurality of vectors 758. Vectors 758 may be substantially similar to vector 550 (Fig. 5) in that vectors 758 represent the difference in location between the actual boundary 730 and the measured boundary 740 in an orthogonal direction to the imaging beam 710. However, while vector 550 of Fig. 5 may represent the direction from the actual boundary to the measured boundary, vectors 758 are opposite. Vectors 758 extend from the measured boundary 740 to the actual boundary 730. In this way, Fig. 7 may illustrate an additional embodiment of the present disclosure. Specifically, the vector denoting displacement from an actual boundary to a measured boundary, as in Fig. 5, may be calculated as *D*(*x*) = *K σ*(*x*), as will be discussed in more detail hereafter. A vector denoting displacement from a measured boundary to an actual boundary, as in Fig. 7, may be calculated as -*D*(*x*) = *-K σ*(*x*). In some embodiments, the vectors 758 and/or vector 550 of Fig. 5 may be referred to as point spread function width-dependent lateral border correction vectors and may be determined at each or any point of the chamber surface both from the measured boundary to the actual boundary and vice versa. Vectors 758 extend only in a lateral, orthogonal, or perpendicular direction to the ultrasound imaging beam 710. As further illustrated in Fig. 7, the magnitudes of vectors 758 depend on the depth of the corresponding location within the patient (e.g., relative to the ultrasound probe) and is directly proportional to the magnitude of the ultrasound point spread function illustrated by Gaussian curves 712, Gaussian714, and Gaussian716.

Similar to the ultrasound point spread function previously discussed, vectors 758 may be defined or modelled as a function dependent on *x*, the distance of a point from the ultrasound probe. For example, any one of vectors 758 may be modelled as a function -D(x), where D(x) may be defined as *D*(*x*) = *K σ* (*x*)*.* The function D(x) may be defined as how far a border detected purely parallel the ultrasound beam would be displaced laterally from the anatomical border. Due to its dependence on *K*, the function D(x) is typically specific to the anatomy and ultrasound acquisition type and can be modelled as a simple multiplier to the value of σ as shown. *σ*(*x*) is representative of the Gaussian function used to model the ultrasound point spread function *PSF*(*x*). *K* may be any suitable constant value. For example, *K* may be a calibrated value dependent on any number of suitable characteristics of or relating to anatomical structure 700, such as the density, mass, volume, orientation, surface continuity, or any other suitable feature of anatomical structure 700, or any liquid or gas within or around anatomical feature 700. In some embodiments, ultrasound imaging system 100 may store constant *K* within memory 138 (Fig. 1), or access constant *K* from a list stored in a separate server, computer, or cloud-based storage device, or any other suitable location. In other embodiments, constant *K* may be calculated or calibrated in a point-of-care setting, before or during an imaging procedure, or at any other suitable time or location. Constant *K* may additionally be manually input to ultrasound imaging system 100 by a system manufacturer or a user or supplied in any other suitable manner. In some embodiments, constant *K* may be calibrated according to the acquisition and anatomy type related to anatomical feature 700 or any other anatomical object measured with ultrasound imaging system 100. Calibration of constant K will be discussed in more detail with respect to Figs. 8 and 9 hereafter.

Additionally, depicted in Fig. 7 is a plurality of correction vectors 768. Correction vectors 768 may be similar to correction vector 668 (Fig. 6). Similar to correction vector 668, correction vectors 768 may represent a distance and direction from measured boundary 740 to actual boundary 730 in a direction normal to the surface of anatomical structure 700. Correction vectors 768 need not be oriented orthogonal to the direction of ultrasound imaging beam 710 because the direction of correction vectors 768 are dictated by the orthogonal direction of the surface of anatomical structure 700 at various locations. The magnitude of correction vector 768 may represent the extent to which the point spreading effect caused by convolving a scatterer map with a point spread function applies in a direction normal to the boundary 730 and represents an approximation of the distance the measured boundary 740 varies from the actual boundary 730. In some embodiments displacement vectors , or vectors extending from an actual boundary to a measured boundary, are of equal but opposite magnitude and direction than correction vectors. For example, correction vectors like vector 660 of Fig. 6 may be calculated as a function *C*(*x*) = (-*N*(*x*) · *D(x))N(x),* where *D*(*x*) may be defined as *D(x)* = *K σ(x)* and is equal to vector 550 of Fig. 5, and *N*(*x*) represents a surface normal vector with respect to actual boundary 730. *C*(*x*) may therefore represent a vector approximating the direction and distance from measured boundary 740 to actual boundary 730 at any point within, along, or around anatomical structure 700. Any suitable form of *C*(*x*) may therefore be applied to measured positions of anatomical structure 700 to provide to a user of ultrasound imaging system 100 correct positions, measurements, and/or other relevant values. In this manner, correction vectors 768 shown in Fig. 7 may be similarly calculated or approximated as *C(x)* = (-*N*(*x*) · *D(x)N(x)* and may denote a direction and magnitude from the measured boundary 740 to the actual boundary 730. This advantageously remedies the inaccuracies inherent within convolved ultrasound imaging applications without the need of deconvolution or other implementations. Because the magnitude and direction of each of the correction vectors 768 , each correction vector 768 may be dependent or based on the depth of the corresponding location of the vectors and/or the orientation of the measured boundary at the corresponding location relative to the ultrasound beam 710.

Additionally, depicted in Fig. 7 is vector 755 and correction vector 765. Vector 755 may be substantially similar to vectors 758 and/or may be included within the set of vectors 758 in that vector 755 extends in a direction perpendicular to the direction of propagation of the ultrasound imaging beam 710 and corresponds to the magnitude of the spreading effect of the ultrasound point spread function as illustrated by Gaussian curves 712, 714, and 716. It is noted that vector 755 is of a very similar but slightly larger magnitude than the vector 758 adjacent to it in Fig. 7 because vector 755 corresponds to a point farther from the position of probe 110 than the adjacent vector 758. However, vector 755 points in the same direction as the adjacent vector 758. By contrast, correction vector 765 is of a magnitude much smaller than the adjacent correction vector 768 and in a different direction. Correction vector 765 may be substantially similar to correction vectors 768 and may be included within the set of correction vectors 768. Correction vector 765 extends in a direction perpendicular to the surface of the anatomical structure 700. The magnitude of correction vector 765 depends on a constant calibrated value associated with the structure, the skew angle of the boundary in relation to the direction of the ultrasound imaging beam 710, and the distance of the location from the origin of the ultrasound imaging beam 710. Although the effect of the ultrasound point spread function is greater at the location of correction vector 765 than at the location of the adjacent correction vector 768, because the boundary 740 at the location of correction vector 765 is substantially more perpendicular to the direction of the ultrasound imaging beam 710 than boundary 740 at the location of the adjacent correction vector 768, correction vector 765 has a smaller magnitude than the adjacent correction vector 768. Similarly, a vector (corresponding to vector 758 or correction vector 768) at a point 770 on or along boundary 740 of structure 700 would have a magnitude of zero or be extremely negligible because boundary 740 at that location is almost completely perpendicular to the direction of ultrasound imaging beam 710. In other words, for a given depth from the ultrasound probe 110, the correction vector 768 and correction vector are larger when the orientation of the measured boundary is more parallel to the ultrasound imaging beam 710 and they are smaller when the orientation of the measured boundary is more perpendicular to the ultrasound imaging beam 710.

Fig. 8 is a diagrammatic view of a display of an ultrasound image 800 created by ultrasound imaging system 100, according to aspects of the present disclosure. Fig. 8 illustrates the ultrasound image 800 in the context of calibrating the constant K value related to a specific anatomical feature (e.g., anatomical structure 700 of Fig. 7 or any other anatomical structure 105 of Fig. 1). The constant *K* and, by dependence, the function *D*(*x*) must typically be calibrated according to the acquisition and anatomy type. In some embodiments, an ultrasound phantom 810 may be used and imaged by ultrasound imaging system 100 as one or more steps or substeps of a method of calibrating a constant Kcorresponding to the anatomical object. For example, in some embodiments, ultrasound phantom 810 may be any suitable ultrasound phantom having characteristics or echogenicity similar to the anatomical object to be imaged by ultrasound imaging system 100, including but not limited to a water phantom, agar phantom, gelatin phantom, guar gum-based phantom, Blue Phantom^{®}, 3-D printed phantoms, and other phantoms. Phantoms may also include natural anatomical phantoms such as a meat phantom of any suitable test subject, including a cadaver. In some embodiments, and as shown in Figs. 8 and 9, the ultrasound phantom 810 may be a hypoechoic egg phantom. In some embodiments, an ultrasound phantom 810 of known volume with any appropriate desirable characteristics may be measured at various depths using the ultrasound imaging system 100 in the same acquisition type and the ultrasound image 800 may be generated. A measured boundary 840 corresponding to the features and structures of ultrasound phantom 810 may be generated and displayed to a user of ultrasound imaging system 100 as illustrated in Fig. 8.

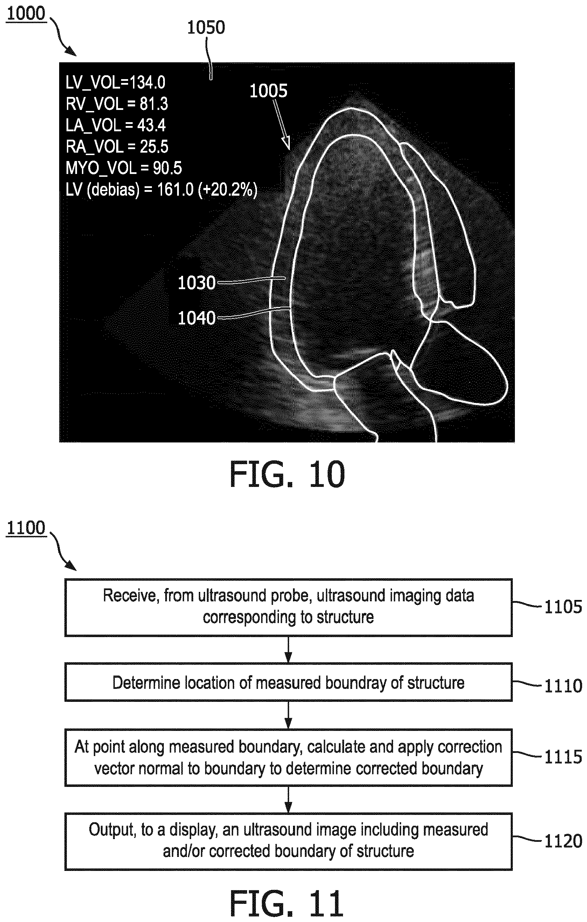

Fig. 9 is diagrammatic view of an enlarged portion of the ultrasound image 800 of Fig. 8, according to aspects of the present disclosure. The ultrasound image 800 in Fig. 9 includes measured boundary 840 and actual boundary 930. To calibrate a constant Kcorresponding to a predetermined anatomical structure, ultrasound imaging system 100 may utilize a least squares optimization process to minimize the difference between the known volume of ultrasound phantom 810 and a volume calculated by ultrasound imaging system 100 based on the actual boundary 930 generated by ultrasound imaging system 100. During this process, ultrasound imaging system 100 may find a value of constant *K* which produces the least error between these two volumes to serve as an approximation of a similar *K* to be applied when imaging anatomical objects with similar characteristics or echogenicity to the ultrasound phantom 810. This K value may then be incorporated into an application which quantifies the volume of a similar structures such as a heart's left ventricle, as shown and further discussed in Fig. 10. It is noted that the least squares minimization process is simply an exemplary method used by ultrasound imaging system 100. Many additional methods of calculating a constant K may be utilized by ultrasound imaging system 100 by comparing known volumes with calculated volumes based on a plurality of perspective actual boundaries 930, including but not limited to ordinary least regression, nonlinear regression, polynomial regression, logistic regression, quantile regression, or any other suitable data fitting or regression analysis method, approximation, or future developed method or algorithm.

Fig. 10 is a diagrammatic view of a screen display including an ultrasound image 1000 created by the ultrasound imaging system 100, according to aspects of the present disclosure. Fig. 10 may further depict an exemplary display of an anatomical structure 1005 as represented by a measured boundary 1040 and an actual boundary 1030. The anatomical structure 1005 shown in Fig. 10 may be any suitable structure included previously. The anatomical structure 1005 depicted in Fig. 10 may be a heart's left ventricle. The actual boundary 1030 can be a corrected boundary that is generated by the ultrasound imaging system to correct errors in the measured boundary 1040. This actual boundary 1030 may be graphically overlaid over the ultrasound image 1000. The actual boundary 1030 may be calculated using a constant K calibrated with a method substantially similar to that described with reference to Fig. 8 and Fig. 9. The anatomical structure 1005 may be substantially similar to anatomical feature 700 or may exhibit similar characteristics including similar acoustic impedances of materials as ultrasound phantom 810 described in Fig. 8 and Fig. 9. In addition, actual boundary 1030 may be exhibit substantially similar characteristics or features as actual boundary 930 of Fig. 9. Measured boundary 1040 may be similar to measured boundary 840 (Figs. 8, 9).

As illustrated in Fig. 10, ultrasound imaging system may display a plurality of lines reflecting boundaries such as actual boundary 1030 and measured boundary 1040 within ultrasound image 1000. These additional lines may correspond to boundaries of or relating to anatomical structure 1005 or may correspond to additional anatomical structures or features surrounding, within, in connection with, or otherwise related to anatomical feature 1005. In some embodiments, ultrasound imaging system 100 may display both lines corresponding to actual boundaries 1030 and lines corresponding to measured boundaries 1040. The ultrasound image 1000 can include a graphical overlay of the actual boundary 1030 and/or the measured boundary 1040. The graphical overlay can visually accentuate the actual boundary 1030 and/or the measured boundary 1040 within the image by identifying and/or highlighting the boundary relative to other anatomical features within the image. In other embodiments, ultrasound imaging system 100 may display to a user only lines corresponding to measured boundaries 1040. In other embodiments, ultrasound imaging system 100 may display to a user only lines corresponding to actual boundaries 1030 and a user may discern a difference between the actual boundary 1030 displayed and features of ultrasound image 1000 indicating a measured boundary.

Any number or type of metrics 1050 may additionally be displayed within or around ultrasound image 1000. The metrics 1050 can include values associated with the measured boundaries 1040 and/or the modified/actual boundary 1030. Metrics 1050 may correspond to one or more volumes relating to significant features, cavities, or structures of or relating to anatomical structure 1005. In addition, dimensions of a structure 1005 or other anatomical object 105 such as length, height, depth, width, circumference, diameter, radius, or other relevant dimension may be displayed to a user. Metrics 1050 may further include any other suitable metric. Metrics 1050 may be displayed overlaid over ultrasound image 1000 as shown in Fig. 10, or may alternatively be displayed to the right, left, above, or beneath ultrasound image 1000.

It is further noted that ultrasound image 1000, and any other ultrasound image, including ultrasound image 800, may be displayed to the user as a video or in video-like format in real time in a point-of-care setting. Alternatively, ultrasound imaging system 100 may capture video comprising a plurality of ultrasound image frames and save images and/or videos within memory 138. Any saved images or videos may further comprise biographical or other information relating to patients or any other suitable information. In other embodiments, ultrasound image 1000, ultrasound image 800, and any other ultrasound image previously mentioned in the present disclosure may be only static images. In some embodiments, although two-dimensional images may be illustrated throughout the present disclosure, data corresponding to three-dimensional images, videos, or models may be captured, stored, saved, and/or analyzed by ultrasound imaging system 100 in substantially the same manner as set forth herein.

Fig. 11 is a flow diagram of a method 1100 which may be employed by ultrasound imaging system 100 to correct inaccuracies in an ultrasound image, according to aspects of the present disclosure. As illustrated, method 1100 includes a number of enumerated steps, but embodiments of method 1100 may include additional steps before, after, or in between the enumerated steps. In some embodiments, one or more of the enumerated steps may be omitted, performed in a different order, or performed concurrently. One or more steps of method 1100 can be carried out by any suitable processor circuit or processing component (e.g., processor circuit 116, processor circuit 134, and/or processor circuit 200).

At step 1105, method 1100 includes receiving, from ultrasound probe 110, ultrasound imaging data corresponding to a structure within a patient. The structure may be substantially similar to structure 105, anatomical structure 700, structure 1005, or any other suitable structure. As previously stated, a structure may be any suitable organ, muscle, tissue, and/or man-made or natural structure within a patient's anatomy. In some embodiments, the method 1100 includes the processor circuit generating an ultrasound image or video based on the ultrasound imaging data.

At step 1110, method 1100 includes determining a location of a measured boundary of the structure to be measured by ultrasound imaging system 100. The measured boundary may be substantially similar to boundaries previously identified in the present application. For example, the measured boundary may be substantially similar to measured boundary 430, measured boundary 740, measured boundary 840, or measured boundary 1040 previously mentioned. In some embodiments, the measured boundary may be any suitable boundary between two media within a patient or within any other structure. The location of the measured boundary may be determined by ultrasound imaging system 100 using methods previously identified in the present disclosure. For example, acoustic waves may be emitted from probe 110 and reflect off of various structures within a patient or in any other suitable environment. Reflected acoustic waves may then be measured by probe 110 or any other equivalent component within an ultrasound imaging system to determine the location of the measured boundary. As noted, the location of the measured boundary will be subject to the blurring effect inherent in the ultrasound imaging point spread function. This effect is observed in all directions perpendicular to the direction of wave propagation of waves emitted by probe 110. This blurring effect increases with depth, as previously discussed, making it difficult to determine the exact location of a measured boundary in an environment imaged by ultrasound imaging system 100.

At step 1115, method 1100 includes calculating and applying a correction vector normal to the measured boundary to determine a corrected boundary at a point along the measured boundary. This correction vector may be substantially similar to correction vector 668. The correction vector 668 represents the distance and direction from a point on a measured boundary back to a point on the actual or corrected boundary. In some embodiments, the correction vector may be calculated based on a surface normal vector relative to the measured boundary, a previously calibrated value corresponding to the acoustic properties of the structure to be measured, the properties of the ultrasound point spread function associated with ultrasound imaging system 100 and/or the environment, and/or the distance from the selected location along the measured boundary from probe 110. In other embodiments, the correction vector may be calculated based on additional variables or measurements or may not require all of the mentioned variables or measurements.

At step 1120, method 1100 includes outputting, to a display, an ultrasound image including the measured boundary and/or the calculated corrected boundary of the structure. A plurality of correction vectors may be applied at any suitable location along a measured boundary within an ultrasound image to create a corrected boundary. The corrected boundary within an ultrasound image may be displayed to a user with a display substantially similar to display 132 of Fig. 1. The corrected boundary may additionally be displayed to a user in any suitable format. For example, corrected boundary may be displayed as a single line extending substantially parallel to an additional line representing the measured boundary. In addition, the lines may be distinguished by using different colors, patterns, such as dotted lines or lines comprising different shapes or patterns of propagation. In some embodiments, a measured boundary may not be displayed. In some embodiments, the measured boundary may be identified by its associated blurred scatterers within an ultrasound image. In some embodiments, the corrected boundary may be displayed as a single line or a plurality of lines which may correspond to ranges of accuracy or certainty or may denote any other suitable characteristic or feature of the boundary within the structure. A wide variety of methods of displaying both the measured boundary and the corrected boundary are fully contemplated and may be used to convey to a user the locations of the corrected boundary and/or the measured boundary.

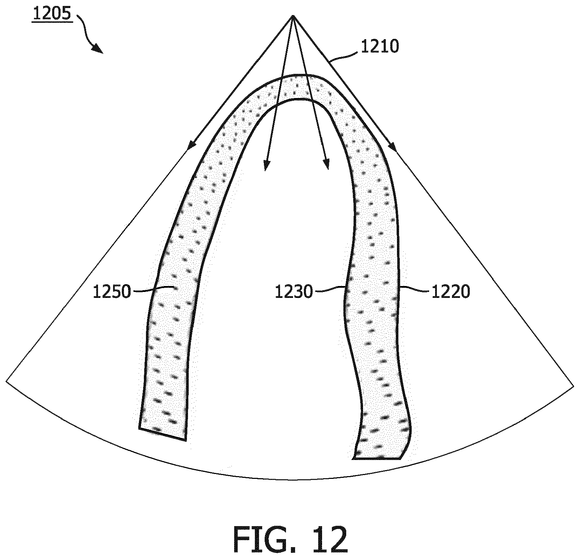

Fig. 12 is a diagrammatic view of an anatomical structure 1205 within a patient displayed in cartesian space, according to aspects of the present disclosure. Anatomical structure 1205 may be substantially similar to previously mentioned structures, including structure 105, anatomical structure 700, structure 1005, or any other suitable structure. Structure 1205 may be any suitable organ, muscle, tissue, natural and/or man-made structure within a patient's anatomy. Fig. 12 depicts a plurality of arrows corresponding to acoustic waves of an ultrasound imaging beam 1210 emitted by the ultrasound probe (e.g., probe 110 of Fig. 1) that is positioned at the top of the image. In some embodiments, ultrasound imaging beam 1210 may propagate in a diverging manner, in different directions from a common point corresponding to the location of the probe. In cartesian space, the ultrasound imaging beams have some angular width so that in the nearfield close to the origin of the imaging beams, the angular width is thin. However, in the far field, farther away from the origin of the imaging beams, the angular width widens considerably resulting in increased spreading farther away from the probe. In some embodiments, an ultrasound imaging beam may propagate in substantially one uniform direction such that arrows corresponding to the beam direction may be parallel to one another. In some embodiments, in cartesian space (Fig. 12), the ultrasound imaging beam appears to be diverging from a common point while in polar space, the same ultrasound imaging beam appears parallel. Fig. 12 further depicts a plurality of points or scatterers 1250. Scatterers 1250 are representative of components within that anatomy that reflect acoustic waves emitted by probe 110. These ultrasound echoes are subsequently received or measured by ultrasound probe. As shown in Fig. 12, the anatomical structure includes an inner boundary 1230 and an outer boundary 1220. Only a portion of the anatomical structure 1205 may be depicted in Fig. 12. For example, a boundary may exist at a location farther from the ultrasound probe than depicted in ultrasound image 1200 (e.g., past the bottom of the image in Fig. 12). This border may be perpendicular to the direction of beam propagation such that anatomical structure 1205 is a substantially closed structure.

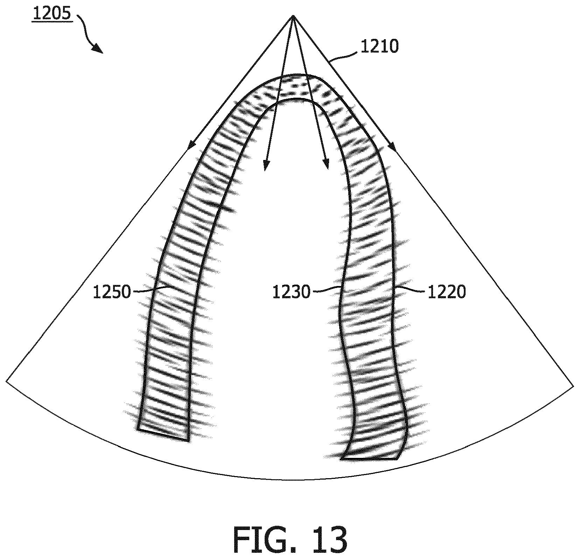

Fig. 13 is a diagrammatic view of the anatomical structure 1205 of Fig. 12 as measured by an ultrasound imaging system, according to aspects of the present disclosure. Like Fig. 12, Fig. 13 also depicts the anatomical structure 1205 in cartesian space. Fig. 13 differs from Fig. 12, however, in that the effect of the ultrasound point spread function is illustrated in the form of scatterers 1250 being blurred. The effect shown in Fig. 13 is generally what is measured by an ultrasound imaging system. The spreading of the scatterers from the perspective of the ultrasound imaging system leads to inaccuracies because it is difficult to ascertain the actual location of boundaries 1220 and 1230 within structure 1205. As described above, because the ultrasound point spread function is depth-dependent, the scatterers 1250 that are located farther from the ultrasound probe (the scatterers 1250 towards the bottom of the image in Fig. 13) are more severely blurred, while scatterers 1250 closer to the ultrasound probe (the scatterers towards the top of the image in Fig. 13) are not as dramatically affected. The present disclosure describes techniques for correcting these inaccuracies so that the actual location of the boundaries 1220 and 1230 can be determined and/or presented to the user.

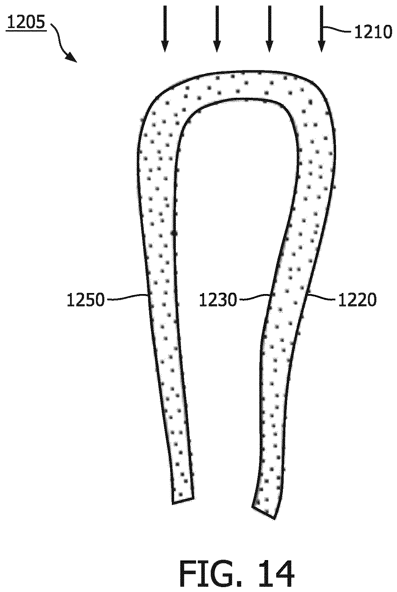

Fig. 14 is a diagrammatic view of the anatomical structure 1205 of Fig. 12 displayed in polar space, according to aspects of the present disclosure. As previously discussed, in polar space, arrows corresponding to ultrasound imaging beam 1210 are substantially parallel to one another such that ultrasound imaging beam 1210 appears to propagate in one uniform direction. Fig. 14 further depicts scatterers 1250 without the previously discussed blurring effect of the ultrasound point spread function.

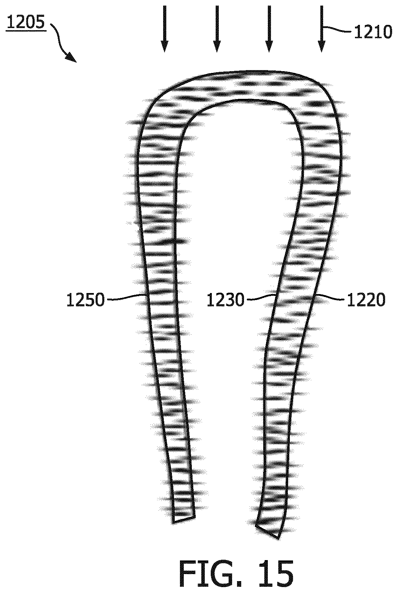

Fig. 15 is a diagrammatic view of the anatomical structure 1205 of Fig. 12, as measured by an ultrasound imaging system and displayed in polar space, according to aspects of the present disclosure. Fig. 15 depicts scatterers 1250 subject to the blurring effect of the ultrasound point spread function, which gives rise to the inaccuracy inherent in determining the location of boundaries 1220 and 1230. Such inaccuracy is readily apparent and more severe for boundaries within a structure which are positioned further away from probe 110 and which extend substantially parallel to the direction of propagation of ultrasound image beam 1210. According to the present disclosure, the ultrasound imaging system 100 provides an improved border detection by calculating and applying one or more correction vectors to boundary 1230 or boundary 1220. This correction may be performed in polar space or cartesian space. Any of the above calculations of vectors, boundaries, or any other metric may be performed in either the cartesian or polar space.

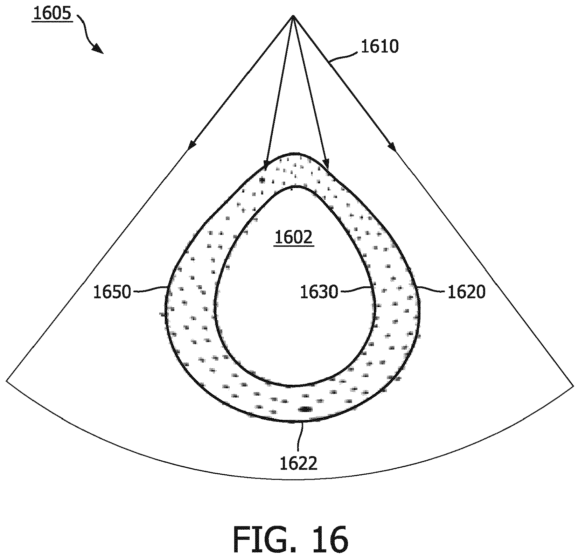

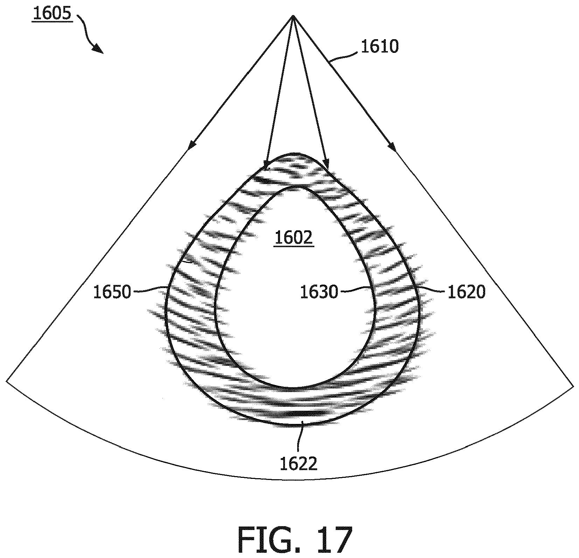

Fig. 16 is a diagrammatic view of an anatomical structure 1605 within a patient displayed in cartesian space, according to aspects of the present disclosure. Anatomical structure 1605 may be substantially similar to previously displayed structures. Anatomical structure 1605 may comprise a substantially closed structure such that it fully encloses and defines a space 1602 within structure 1605. Fig. 16 also depicts a plurality of scatterers 1650. The anatomical structure 1605 includes an inner boundary 1630 and an outer boundary 1620. Anatomical structure 1605 includes a point or location 1622 at a location furthest away from ultrasound probe at which boundary 1630 and boundary 1620 extend in directions perpendicular to the direction of propagation of ultrasound imaging beam 1610 as will be discussed in more detail with reference to Fig. 17.

Fig. 17 is a diagrammatic view of the anatomical structure 1605 of Fig. 16, as measured by ultrasound imaging system 100 and displayed in cartesian space, according to aspects of the present disclosure. Fig. 17 depicts scatterers 1650 subject to the blurring effect of the ultrasound point spread function, which gives rise to the inaccuracy inherent in determining the location of boundaries 1620, 1630. Such inaccuracy is readily apparent and more severe for boundaries within a structure which are positioned further away from probe (at the top of image in Fig. 16) and which extend substantially parallel to the direction of propagation of ultrasound image beam 1610. However, at location 1622 and at any similar locations along a boundary of a structure in which a boundary of a structure extends in any direction perpendicular to the direction of propagation of ultrasound imaging beam 1610, the inaccuracy of determining the location of the boundary is negligible to non-existent. For example, at location 1622, though scatterers 1650 are still subject to the ultrasound point spread function such that they are blurred, because the blurring occurs only in directions perpendicular to the direction of beam propagation and because the boundary extends in this same direction, no blurring occurs in the direction of beam propagation. At such locations, although ultrasound imaging system 100 may still calculate and apply a correction vector to measured boundary 1630 or 1620, the magnitude of the correction vector will be zero or at most negligible. Fig. 17 therefore illustrates the ability of ultrasound image system 100 to more accurately determine the location of the actual boundary within a structure of a patient by making a location specific correction at any point along the surface of the boundary. This correction accounts for the distance of the location from the probe and the skew angle of the boundary in relation to the direction of beam propagation. This correction then yields significantly more accurate measurements of any number of metrics obtained in an ultrasound examination.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ultrasound imaging system (100), comprising:
a processor circuit (134, 200) configured for communication with an ultrasound probe (110), the processor circuit configured to:
receive, from the ultrasound probe, ultrasound data representative of an ultrasound beam (305, 710, 1210, 1610) imaging an anatomical structure (300, 700, 1005, 1205, 1605);
determine, based on the ultrasound data, a measured boundary (430, 740, 1040) of the anatomical structure, wherein the measured boundary includes a plurality of locations;
determine a plurality of correction vectors (668, 768) corresponding to the plurality of locations of the measured boundary, wherein a magnitude of a respective correction vector is based on at least one of:
a depth of a corresponding location relative to the ultrasound probe; or
an orientation of the measured boundary at the corresponding location relative to the ultrasound beam;
apply the plurality of correction vectors to the plurality of locations of the measured boundary to determine a corrected boundary (330, 730, 1030); and
output, to a display (132) in communication with the processor circuit, an ultrasound image (800, 1000) based on the ultrasound data, wherein the ultrasound image includes the corrected boundary; and
being **characterized in that**
the plurality of correction vectors are configured to correct an effect of a point spread function of the ultrasound imaging system.

2. The system (100) of claim 1, wherein a direction of the plurality of correction vectors (668, 768) is at least one of normal to the measured boundary (430, 740, 1040) or normal to the corrected boundary (330, 730, 1030).

3. The system (100) of claim 1, wherein the processor circuit (134, 200) is configured to model the point spread function as a Gaussian function.

4. The system (100) of claim 1, wherein the magnitude of the respective correction vector (668, 768) is based on the depth of the corresponding location relative to the ultrasound probe (110) and the orientation of the measured boundary (430, 740, 1040) at the corresponding location relative to the ultrasound beam (305, 710, 1210, 1610).

5. The system (100) of claim 4, wherein the magnitude of the respective correction vector (668, 768), for a given orientation of the measured boundary (430, 740, 1040) at the corresponding location, is larger when the corresponding location is at a larger depth relative to the ultrasound probe (110) and smaller when the corresponding location is at a smaller depth relative to the ultrasound probe.

6. The system (100) of claim 4, wherein the magnitude of the respective correction vector (668, 768), for a given depth of the corresponding location relative to the ultrasound probe (110), is larger when the orientation of the measured boundary (430, 740, 1040) is parallel to the ultrasound beam (305, 710, 1210, 1610) and smaller when the orientation of the measured boundary is perpendicular to the ultrasound beam.

7. The system (100) of claim 1, wherein the plurality of correction vectors (668, 768) is further based on a calibrated value corresponding to one or more characteristics of the anatomical structure (300, 700, 1005, 1205, 1605).

8. The system (100) of claim 1, wherein the processor circuit (134, 200) is further configured to calculate, based on the corrected boundary (330, 730, 1030), a metric (1050) associated with the anatomical structure (300, 700, 1005, 1205, 1605).

9. The system (100) of claim 8, wherein the processor circuit (134, 200) is configured to output the calculated metric (1050) to the display (132).

10. The system (100) of claim 8, wherein the metric (1050) comprises a volume of the anatomical structure (300, 700, 1005, 1205, 1605).

11. The system (100) of claim 1, wherein the processor circuit (134, 200) is further configured to output the measured boundary (430, 740, 1040) to the display (132).

12. The system (100) of claim 1, wherein the corrected boundary (330, 730, 1030) comprises a graphical overlay on the ultrasound image (800, 1000).

13. The system (100) of claim 1, wherein a direction of the plurality of correction vectors (668, 768) is:
inward relative to the measured boundary (430, 740, 1040) when the anatomical structure (300, 700, 1005, 1205, 1605) comprises a hyperechoic chamber; and
outward relative to the measured boundary when the anatomical structure comprises a hypoechoic chamber.

14. The system (100) of claim 1, further comprising:
the ultrasound probe (110).

15. An ultrasound imaging method (1100), comprising:
receiving (1105), at a processor circuit (134, 200) in communication with an ultrasound probe (110), ultrasound data representative of an ultrasound beam (305, 710, 1210, 1610) imaging an anatomical structure (300, 700, 1005, 1205, 1605);
determining (1110), by the processor circuit, a measured boundary (430, 740, 1040) of the anatomical structure based on the ultrasound data, wherein the measured boundary includes a plurality of locations;
determining (1115), by the processor circuit, a plurality of correction vectors (668, 768) corresponding to the plurality of locations of the measured boundary,
wherein a magnitude of a respective correction vector is based on at least one of:
a depth of a corresponding location relative to the ultrasound probe; or
an orientation of the measured boundary at the corresponding location relative to the ultrasound beam;
applying (1115), by the processor circuit, the plurality of correction vectors to the plurality of locations of the measured boundary to determine a corrected boundary (330, 730, 1030); and
outputting (1120), to a display (132) in communication with the processor circuit, an ultrasound image (800, 1000) based on the ultrasound data, wherein the ultrasound image includes the corrected boundary; and
being **characterized in that**
the plurality of correction vectors are configured to correct an effect of a point spread function of the ultrasound imaging system.

## Patentansprüche

1. Ultraschallbildgebungssystem (100), umfassend:
eine Prozessorschaltung (134, 200), die für Kommunikation mit einer Ultraschallsonde (110) konfiguriert ist, wobei die Prozessorschaltung für Folgendes konfiguriert ist, um:
von der Ultraschallsonde für einen Ultraschallstrahl (305, 710, 1210, 1610), der eine anatomische Struktur (300, 700, 1005, 1205, 1605) abbildet, repräsentative Ultraschalldaten zu empfangen;
basierend auf den Ultraschalldaten eine gemessene Grenze (430, 740, 1040) der anatomischen Struktur zu bestimmen, wobei die gemessene Grenze eine Vielzahl von Stellen beinhaltet;
eine Vielzahl von Korrekturvektoren (668, 768), die der Vielzahl von Stellen der gemessenen Grenze entsprechen, zu bestimmen, wobei eine Größe eines jeweiligen Korrekturvektors auf mindestens einem basiert von:
einer Tiefe einer entsprechenden Stelle in Bezug zur Ultraschallsonde; oder
einer Ausrichtung der gemessenen Grenze an der entsprechenden Stelle in Bezug zum Ultraschallstrahl;
die Vielzahl von Korrekturvektoren auf die Vielzahl von Stellen der gemessenen Grenze anzuwenden, um eine korrigierte Grenze (330, 730, 1030) zu bestimmen; und
an eine Anzeige (132) in Kommunikation mit der Prozessorschaltung ein Ultraschallbild (800, 1000) basierend auf den Ultraschalldaten auszugeben, wobei das Ultraschallbild die korrigierte Grenze beinhaltet; und
**dadurch gekennzeichnet, dass**
die Vielzahl von Korrekturvektoren konfiguriert ist, um einen Effekt einer Punktspreizfunktion des zu korrigieren.

2. System (100) nach Anspruch 1, wobei eine Richtung der Vielzahl von Korrekturvektoren (668, 768) mindestens eines von normal zur gemessenen Grenze (430, 740, 1040) oder normal zur korrigierten Grenze (330, 730, 1030) ist.

3. System (100) nach Anspruch 1, wobei die Prozessorschaltung (134, 200) konfiguriert ist, um die Punktspreizfunktion als eine Gauß-Funktion zu modellieren.

4. System (100) nach Anspruch 1, wobei die Größe des jeweiligen Korrekturvektors (668, 768) auf der Tiefe der entsprechenden Stelle in Bezug zur Ultraschallsonde (110) und der Ausrichtung der gemessenen Grenze (430, 740, 1040) an der entsprechenden Stelle in Bezug zum Ultraschallstrahl (305, 710, 1210, 1610) basiert.

5. System (100) nach Anspruch 4, wobei die Größe des jeweiligen Korrekturvektors (668, 768) für eine gegebene Ausrichtung der gemessenen Grenze (430, 740, 1040) an der entsprechenden Stelle größer ist, wenn sich die entsprechende Stelle in Bezug zur Ultraschallsonde (110) in einer größeren Tiefe befindet, und kleiner ist, wenn sich die entsprechende Stelle in Bezug zur Ultraschallsonde in einer geringeren Tiefe befindet.

6. System (100) nach Anspruch 4, wobei die Größe des jeweiligen Korrekturvektors (668, 768) für eine gegebene Tiefe der entsprechenden Stelle in Bezug zur Ultraschallsonde (110) größer ist, wenn die Ausrichtung der gemessenen Grenze (430, 740, 1040) parallel zum Ultraschallstrahl (305, 710, 1210, 1610) ist, und kleiner ist, wenn die Ausrichtung der gemessenen Grenze senkrecht zum Ultraschallstrahl ist.

7. System (100) nach Anspruch 1, wobei die Vielzahl von Korrekturvektoren (668, 768) weiter auf einem kalibrierten Wert basiert, der einer oder mehreren Eigenschaften der anatomischen Struktur (300, 700, 1005, 1205, 1605) entspricht.

8. System (100) nach Anspruch 1, wobei die Prozessorschaltung (134, 200) weiter konfiguriert ist, um basierend auf der korrigierten Grenze (330, 730, 1030) eine Metrik (1050) zu berechnen, die mit der anatomischen Struktur (300, 700, 1005, 1205, 1605) verknüpft ist.

9. System (100) nach Anspruch 8, wobei die Prozessorschaltung (134, 200) konfiguriert ist, um die berechnete Metrik (1050) an die Anzeige (132) auszugeben.

10. System (100) nach Anspruch 8, wobei die Metrik (1050) ein Volumen der anatomischen Struktur (300, 700, 1005, 1205, 1605) umfasst.

11. System (100) nach Anspruch 1, wobei die Prozessorschaltung (134, 200) weiter konfiguriert ist, um die gemessene Grenze (430, 740, 1040) an die Anzeige (132) auszugeben.

12. System (100) nach Anspruch 1, wobei die korrigierte Grenze (330, 730, 1030) eine grafische Überlagerung auf dem Ultraschallbild (800, 1000) umfasst.

13. System (100) nach Anspruch 1, wobei eine Richtung der Vielzahl von Korrekturvektoren (668, 768) ist:
einwärts in Bezug zur gemessenen Grenze (430, 740, 1040), wenn die anatomische Struktur (300, 700, 1005, 1205, 1605) eine echoreiche Kammer umfasst; und
auswärts in Bezug zur gemessenen Grenze, wenn die anatomische Struktur eine echoarme Kammer umfasst.

14. System (100) nach Anspruch 1, weiter umfassend:
die Ultraschallsonde (110).

15. Ultraschallbildgebungsverfahren (1100), umfassend:
Empfangen (1105), an einer Prozessorschaltung (134, 200) in Kommunikation mit einer Ultraschallsonde (110), von für einen Ultraschallstrahl (305, 710, 1210, 1610), der eine anatomische Struktur (300, 700, 1005, 1205, 1605) abbildet, repräsentativen Ultraschalldaten;
Bestimmen (1110), durch die Prozessorschaltung, einer gemessenen Grenze (430, 740, 1040) der anatomischen Struktur basierend auf den Ultraschalldaten, wobei die gemessene Grenze eine Vielzahl von Stellen beinhaltet;
Bestimmen (1115), durch die Prozessorschaltung, einer Vielzahl von Korrekturvektoren (668, 768), die der Vielzahl von Stellen der gemessenen Grenze entsprechen, wobei eine Größe eines jeweiligen Korrekturvektors auf mindestens einem basiert von:
einer Tiefe einer entsprechenden Stelle in Bezug zur Ultraschallsonde; oder
einer Ausrichtung der gemessenen Grenze an der entsprechenden Stelle in Bezug zum Ultraschallstrahl;
Anwenden (1115), durch die Prozessorschaltung, der Vielzahl von Korrekturvektoren auf die Vielzahl von Stellen der gemessenen Grenze, um eine korrigierte Grenze (330, 730, 1030) zu bestimmen; und
Ausgeben (1120), an eine Anzeige (132) in Kommunikation mit der Prozessorschaltung eines Ultraschallbilds (800, 1000) basierend auf den Ultraschalldaten, wobei das Ultraschallbild die korrigierte Grenze beinhaltet; und
**dadurch gekennzeichnet, dass**
die Vielzahl von Korrekturvektoren konfiguriert ist, um einen Effekt einer Punktspreizfunktion des Ultraschallbildgebungssystems zu korrigieren.

## Revendications

1. Système d'imagerie par ultrasons (100) comprenant :
un circuit processeur (134, 200) configuré pour communiquer avec une sonde ultrasonore (110), le circuit processeur étant configuré pour :
recevoir, à partir de la sonde ultrasonore, des données ultrasonores représentatives d'un faisceau ultrasonore (305, 710, 1210, 1610) imageant une structure anatomique (300, 700, 1005, 1205, 1605) ;
déterminer, sur la base des données ultrasonores, une limite mesurée (430, 740, 1040) de la structure anatomique, dans lequel la limite mesurée inclut une pluralité d'emplacements ;
déterminer une pluralité de vecteurs de correction (668, 768) correspondant à la pluralité d'emplacements de la limite mesurée, dans lequel une grandeur d'un vecteur de correction respectif est basée sur au moins l'une de :
une profondeur d'un emplacement correspondant par rapport à la sonde ultrasonore ; ou
une orientation de la limite mesurée à l'emplacement correspondant par rapport au faisceau ultrasonore ;
appliquer la pluralité de vecteurs de correction à la pluralité d'emplacements de la limite mesurée pour déterminer une limite corrigée (330, 730, 1030) ; et
produire en sortie, sur un écran (132) en communication avec le circuit processeur, une image ultrasonore (800, 1000) basée sur les données ultrasonores, dans lequel l'image ultrasonore inclut la limite corrigée ; et
étant **caractérisé en ce que**
la pluralité de vecteurs de correction sont configurés pour corriger un effet d'une fonction d'étalement ponctuel du système d'imagerie par ultrasons.

2. Système (100) selon la revendication 1, dans lequel une direction de la pluralité de vecteurs de correction (668, 768) est au moins l'une d'une perpendiculaire à la limite mesurée (430, 740, 1040) ou d'une perpendiculaire à la limite corrigée (330, 730, 1030).

3. Système (100) selon la revendication 1, dans lequel le circuit processeur (134, 200) est configuré pour modéliser la fonction d'étalement ponctuel comme une fonction gaussienne.

4. Système (100) selon la revendication 1, dans lequel la grandeur du vecteur de correction respectif (668, 768) est basée sur la profondeur de l'emplacement correspondant par rapport à la sonde ultrasonore (110) et l'orientation de la limite mesurée (430, 740, 1040) à l'emplacement correspondant par rapport au faisceau à ultrasons (305, 710, 1210, 1610).

5. Système (100) selon la revendication 4, dans lequel la grandeur du vecteur de correction respectif (668, 768), pour une orientation donnée de la limite mesurée (430, 740, 1040) à l'emplacement correspondant, est plus grande lorsque l'emplacement correspondant est à une plus grande profondeur par rapport à la sonde ultrasonore (110) et plus petite lorsque l'emplacement correspondant est à une plus petite profondeur par rapport à la sonde ultrasonore.

6. Système (100) selon la revendication 4, dans lequel la grandeur du vecteur de correction respectif (668, 768), pour une profondeur donnée de l'emplacement correspondant par rapport à la sonde ultrasonore (110), est plus grande lorsque l'orientation de la limite mesurée (430, 740, 1040) est parallèle au faisceau ultrasonore (305, 710, 1210, 1610) et plus petite lorsque l'orientation de la limite mesurée est perpendiculaire au faisceau ultrasonore.

7. Système (100) selon la revendication 1, dans lequel la pluralité de vecteurs de correction (668, 768) sont en outre basés sur une valeur étalonnée correspondant à une ou plusieurs caractéristiques de la structure anatomique (300, 700, 1005, 1205, 1605).

8. Système (100) selon la revendication 1, dans lequel le circuit processeur (134, 200) est en outre configuré pour calculer, sur la base de la limite corrigée (330, 730, 1030), une mesure (1050) associée à la structure anatomique (300, 700, 1005, 1205, 1605).

9. Système (100) selon la revendication 8, dans lequel le circuit processeur (134, 200) est configuré pour délivrer la mesure calculée (1050) à l'écran (132).

10. Système (100) selon la revendication 8, dans lequel la mesure (1050) comprend un volume de la structure anatomique (300, 700, 1005, 1205, 1605).

11. Système (100) selon la revendication 1, dans lequel le circuit processeur (134, 200) est en outre configuré pour délivrer en sortie la limite mesurée (430, 740, 1040) à l'écran (132).

12. Système (100) selon la revendication 1, dans lequel la limite corrigée (330, 730, 1030) comprend une superposition graphique sur l'image ultrasonore (800, 1000).

13. Système (100) selon la revendication 1, dans lequel une direction de la pluralité de vecteurs de correction (668, 768) est :
vers l'intérieur par rapport à la limite mesurée (430, 740, 1040) lorsque la structure anatomique (300, 700, 1005, 1205, 1605) comprend une chambre hyperéchogène ; et
vers l'extérieur par rapport à la limite mesurée lorsque la structure anatomique comprend une chambre hypoéchogène.

14. Système (100) selon la revendication 1, comprenant en outre :
la sonde ultrasonore (110).

15. Procédé d'imagerie par ultrasons (1100) comprenant :
la réception (1105), au niveau d'un circuit processeur (134, 200) en communication avec une sonde ultrasonore (110), de données ultrasonores représentatives d'un faisceau ultrasonore (305, 710, 1210, 1610) imageant une structure anatomique (300, 700, 1005, 1205, 1605) ;
la détermination (1110), par le circuit processeur, d'une limite mesurée (430, 740, 1040) de la structure anatomique sur la base des données ultrasonores, dans lequel la limite mesurée inclut une pluralité d'emplacements ;
la détermination (1115), par le circuit processeur, d'une pluralité de vecteurs de correction (668, 768) correspondant à la pluralité d'emplacements de la limite mesurée, dans lequel une grandeur d'un vecteur de correction respectif est basée sur au moins l'une de :
une profondeur d'un emplacement correspondant par rapport à la sonde ultrasonore ; ou
une orientation de la limite mesurée à l'emplacement correspondant par rapport au faisceau ultrasonore ;
l'application (1115), par le circuit processeur, de la pluralité de vecteurs de correction à la pluralité d'emplacements de la limite mesurée pour déterminer une limite corrigée (330, 730, 1030) ; et
la production en sortie (1120), à un écran (132) en communication avec le circuit processeur, d'une image ultrasonore (800, 1000) basée sur les données ultrasonores, dans lequel l'image ultrasonore inclut la limite corrigée ; et
étant **caractérisé en ce que**
la pluralité de vecteurs de correction sont configurés pour corriger un effet d'une fonction d'étalement ponctuel du système d'imagerie par ultrasons.
